# EUROPÄISCHE PATENTANMELDUNG

(11) **EP 4 393 322 A2**
(43) Veröffentlichungstag der Anmeldung: **03.07.2024**
(21) Anmeldenummer: 24168975.1
(22) Anmeldetag: 02.12.2020
(51) Int. Cl.: A23L 33/135

(54) **VERFAHREN ZUR UMRÖTUNG VON FLEISCHWAREN**

(30) Priorität: 02.12.2019 DE 102019132648
(62) Teilanmeldung aus: 20020583.9
(71) Anmelder: world wide WIBERG GmbH, 5020 Salzburg (AT)
(72) Erfinder: Alpert, Carl-Alfred, 49080 Osnabrück (DE); Neddermann, Tobias, 32602 Vlotho (DE); Höfler, Kristina, 84489 Burghausen (DE)
(74) Vertreter: Schneiders & Behrendt Bochum

(57) **Zusammenfassung**

Verfahren zur Umrötung von Fleischerzeugnissen unter Verwendung von Mikroorganismen, wobei Fleischmasse mit bis zu 1x 10¹² Kolonie bildenden Einheiten pro kg Fleischmasse einer lebensmitteltauglichen Spezies der Gattungen Staphylococcus oder Kocuria, die in der Lage sind, Arginin in Stickstoffmonoxid zu überführen, und mit bis zu 10 g pro kg Fleischmasse eines Arginin enthaltenden Substrates auf pflanzlicher oder Hefebasis versetzt wird und einem definierten Umröteprozess unterzogen wird.

## Beschreibung

Die Erfindung betrifft ein Verfahren zur Umrötung von Fleischerzeugnissen unter Verwendung von Mikroorganismen sowie eine Mischung zur Durchführung dieses Verfahrens und neue Mikroorganismen der Gattungen Staphylococcus und Kocuria.

Bei den herkömmlichen Verfahren zur Umrötung von Fleischerzeugnissen werden diesen Nitritpökelsalz und weitere übliche Zusatzstoffe oder auch nitrathaltige Zutaten zugesetzt. Durch das Zusammenwirken von Kochsalz, Nitrit oder anderen Nitratquellen, Pökelhilfsstoffen, fleischeigenen Enzymen und der Pökelflora bewirkt dieser Zusatz die Reifung, Konservierung und auch die Umrötung des Fleischerzeugnisses, sowie die Stabilisierung der Fleischfarbe, die Entwicklung eines typischen Aromas und nimmt Einfluss auf die Fleischbeschaffenheit. Die Umrötung kann je nach Verfahren bei der Herstellung von Brühwurst einige Stunden in Anspruch nehmen.

Diese Pökelung mit anderen Nitratquellen kann bei Rohwurstwaren zu Produkten mit stabilerer Pökelfarbe und einem volleren Aroma führen. Sie erfordert aber längere Reifungszeiten und ist mikrobiologisch riskanter. Die Reifungszeit kann bei großteiligen Einzelstücken und langzeitgereiften Rohwürsten länger als vier Wochen dauern.

Bei den heute vorwiegend durchgeführten Verfahren der normalen Umrötung bei Brühwurst und Kochpökelwaren, wird nahezu ausschließlich mit Nitritpökelsalz gearbeitet. Den so hergestellten Lebensmittel werden zwischen 80 und 160 ppm Nitrit zugesetzt um die gewünschte Pökelfarbe und das typische Pökelaroma zu erreichen. Bei derartig hergestellten Brühwürsten und Kochpökelwaren muss man mit relativ hohen Rest-Nitrit- und Nitratmengen rechnen. Diese Rest-Nitrit- und Nitratmengen, können ohne weiteres mehr als 40 ppm betragen.

Aufgabe der Erfindung ist daher die Bereitstellung eines Verfahrens zur Umrötung von Fleischerzeugnissen, das schnell und effizient verläuft und ohne die Verwendung von Nitrat/Nitritpökelsalz oder anderen Nitrit- oder Nitratquellen durchgeführt werden kann. Ein weiterhin verfolgtes Ziel ist es die potenziell karzinogenen Rest-Nitrit- und Nitratmengen auf Mengen deutlich unterhalb der traditionell erreichten Werte zu reduzieren
Diese Aufgabe wird in ein Verfahren der eingangsgenannten Art gelöst, bei dem die Fleischmasse mit bis zu 1 x 10¹² Kolonie bildenden Einheiten (KbE) pro kg Fleischmasse einer oder mehrerer lebensmitteltauglichen Spezies der Gattungen Staphylococcus oder Kocuria, die in der Lage sind, Arginin in Stickstoffmonoxid (NO) zu überführen, und einem Arginin enthaltenden Substrat auf pflanzlicher, tierischer, mikrobieller oder Pilz-, insbesondere Hefebasis versetzt wird und einem definierten Umröteprozess unterzogen wird (industrieübliche Delta T Kochung oder vergleichbar).

Bei dem erfindungsgemäßen Verfahren wird die Umrötung der Fleischerzeugnisse über Mikroorganismen herbeigeführt, die in der Lage sind, Arginin in NO zu überführen. Das NO reagiert sofort ohne Akkumulation in dem Fleischerzeugnis mit Myoglobin zu Nitrosomyoglobin, das nach Erhitzung oder Trocknung (Eiweißdenaturierung) eine stabile Rotfärbung erzeugt, und verbleibt nicht im Fleisch.

Das NO stammt letztlich aus der Aminosäure Arginin des Substrates, die aber nicht als alleiniges Substrat dienen kann. Nur mit Arginin kann der Prozess nicht gestartet werden. Vom Stoffwechsel her gesehen kommt das NO aus Arginin und Sauerstoff über eine Stickoxidsynthase unter Abspaltung von Citrullin.

Die Reaktion lässt sich durch Zugabe von Aspartat als freie Säure und Aminosäure enthaltenden Substraten, wie Eiweißpulver oder Sojaproteinisolat, signifikant verstärken.

Allerdings sind Staphylokokken und Kocurien nicht ohne Weiteres in der Lage, Arginin in NO umzuwandeln; es bedarf hierzu Arginin enthaltende Substrate auf pflanzlicher, tierischer, mikrobieller oder Pilz-, insbesondere Hefebasis. Zur Auswahl solcher Quellen wurde ein Schnelltest entwickelt, der die Eignung aufzeigt und im Weiteren beschrieben ist.

Die Mikroorganismen werden der Fleischmasse in einer Menge von bis zu 1 x 10¹² Kolonie bildenden Einheiten (KbE) pro kg Fleischmasse zugesetzt, insbesondere in einer Menge von etwa 1 x 10¹¹ KbE. Diese Menge hat sich in der Regel als völlig ausreichend für eine rasche Umrötung der Fleischerzeugnisse erwiesen.

Bei den Substraten handelt es sich um Arginin enthaltende pflanzliche Substrate oder Hefeprodukte. Besonders geeignet sind pflanzliche oder tierische Peptone, mikrobielle Extrakte sowie Pilzpulver und Hefeextrakte, die den oben erwähnten Schnelltest bestehen. Welche Bestandteile dieser Substrate geeignet sind, die Mikroorganismen dazu anzuregen, die Umwandlung von Arginin in NO zu bewirken, ist nicht bekannt, die Auswahl der Substrate durch den Schnelltest hat sich aber als zuverlässig erwiesen.

Geeignete Substrate auf Hefebasis sind Hefeextrakte, wie sie im Handel erhältlich sind. Als pflanzliche Substrate kommen insbesondere Pflanzenextrakte von Erbse, Kartoffel oder Soja infrage, vorzugsweise in Form von Peptonen. Als tierische Substrate kommen insbesondere Extrakte aus Fleisch oder tierischen Erzeugnissen wie Milch in Frage, vorzugsweise In Form von Peptonen. Als Substrate auf Pilzbasis kommen Pilzpulver oder Pilzextrakte in Frage, zum Beispiel von Champignons, Steinpilzen oder Shiitake-Pilzen. Als mikrobielle Substrate kommen Zellextrakte oder hdrolysierte Zellen von zum Beispiel Lactobazillen in Frage. Die Substrate werden in einer Menge von bis zu 20 g, vorzugsweise 1 bis 10 g, insbesondere in einer Menge von 2,5 g bis 6 g pro kg Fleischmasse eingesetzt. Bevorzugt sind Mengen von bis zu 10g, besonderes 2 g bis 6 g und insbesondere 2,5 g bis 3,5 pro kg Fleischmasse.

Die Umrötung der Fleischerzeugnisse erfolgt in der Regel bei einer Temperatur von bis zu 60°C, vorzugsweise bis zu 40°C, über eine Zeit von bis zu 4 h. In der Praxis wird das erfindungsgemäß vorbereitete Fleischerzeugnis nach dem Vermengen der Bestandteile und Kuttern zunächst innerhalb von 2 h auf eine Temperatur von 40°C bis 60°C erwärmt und anschließend auf eine Kerntemperatur von bis zu 80°C gebracht. Die Reifung und Umwandlung des Arginin in NO erfolgt innerhalb dieser Erwärmungsphase auf bis zu 60°C. Damit ist der Reifeprozess in sehr kurzer Zeit abgeschlossen, ebenso für Kochpökel- und Rohpökelwaren.

Als besonders geeignet haben sich eine Reihe von Stämmen gezeigt, die nach den Bestimmungen des Budapester Vertrags bei der DSMZ hinterlegt wurden. Es handelt sich um die Stämme:
Staphylococcus xylosus FASP 2 und FASP 3,
Staphylococcus vitulinus FASP 4,
Staphylococcus equorum FASP 1 und FASP 5,
Kocuria salsicia FASP 6,
Kocuria varians FASP 7 und
Staphylococcus carnosus FASP 8.

Es ist davon auszugehen, dass zahlreiche weitere Stämme der Gattungen Staphylococcus und Kocuria die Fähigkeit haben, unter den hier aufgezeigten Verfahrensbedingungen Arginin aus Substraten pflanzlicher, tierischer, mikrobieller oder Pilz-, insbesondere Hefebasis in NO umzuwandeln.

Als Einsatzgebiete für das erfindungsgemäße Verfahren kommen insbesondere Brüh- und Kochpökelwaren infrage. Es handelt sich dabei um Brühwurst, Brühdauerwurst, Hot Dogs, Kochschinken und dergleichen. Das Verfahren kann auch für die Herstellung von Rohwürsten herangezogen werden und in anderen Einsatzgebieten, in denen derzeit Nitritpökelsalz oder Ersatzstoffe wie beispielsweise andere Nitratquellen verwandt werden, eingesetzt werden.

Die Erfindung betrifft ferner Mikroorganismen der oben genannten Gattungen und ihre Verwendung bei der Umrötung von Fleischerzeugnissen zusammen mit Arginin enthaltenden Substraten auf pflanzlicher oder Hefebasis sowie Packungen zur Umrötung von Fleischerzeugnissen, die einen oder mehrere Stämme einer oder mehrerer Spezies der Gattung Staphylococcus oder Kocuria, die in der Lage sind, Arginin in Gegenwart eines Substrates, etwa auf pflanzlicher oder Hefebasis, in Stickstoffmonoxid überführen, in gefriergetrockneter oder anderweitig konservierter Form sowie gesondert davon ein Arginin enthaltendes Substrat auf pflanzlicher, tierischer, mikrobieller oder Pilz-, insbesondere Hefebasis enthalten.

### Schnelltest

Die Eignung eines pflanzlichen Substrates oder eines Hefe Extraktes kann mit dem nachfolgend beschriebenen Schnelltest ermittelt werden.

Die zu testende Quelle wird in wässrige Lösung gebracht und mit einer kleinen Menge eines Stammes von Staphylococcus equorum, der sich als geeignet für die Umwandlung von Arginin in NO erwiesen hat, beispielsweise FASP 1-8, Phosphatpuffer und Glukose versetzt. Die Lösung wird 2 h bei 30°C inkubiert. Anschließend werden 0,5 ml der Lösung mit 0,5 ml Griess-Reagenz versetzt und diese Lösung bei 540 nm fotometrisch vermessen. Liegt der Wert über 0,35, besser über 0,7, noch besser über 1,0 und insbesondere über 1,5, ist der Zusatzstoff verfahrensgemäß geeignet. Bei einem Wert unter 0,35 ist keine ausreichende Funktion gegeben.

### Beispiel

50 kg Schweinefleisch II, 3 mm, 15 kg Schweinebacken, 3 mm, 10 kg Speck, 3 mm und 25 kg Eis werden miteinander gewolft mit 18 g/kg Kochsalz, 3g/kg Phosphat und Ascorbinsäure versetzt, gekuttert und anschließend mit 0,1 g entsprechend 1x 10¹¹ KbE Staphylococcus equorum FASP 5 und 3g/kg Hefeextrakt versetzt. Das Brät wird in Sterildärme mit Kaliber 90 gefüllt und in die Kochkammer gefahren. Dort wird das Brät 2 h bei 40°C Kammertemperatur umröten gelassen und dann bei 78°C Kammertemperatur bis zur Erreichung einer Kerntemperatur von 72°C erhitzt. Die Kerntemperatur wurde nach 2:20 h erreicht. Anschließend werden die Würste kalt abgeduscht und abkühlen gelassen.

### Vergleichsbeispiel

Das Beispiel wurde unter Verwendung von 18 g/kg Nitritpökelsalz wiederholt, wobei auf den Zusatz von Staphylococcus equorum und Hefeextrakt verzichtet wurde. Reifung und Brühen erfolgt nach Standardbedingungen.

Die nach dem Beispiel und Vergleichsbeispiel erhaltenen Produkte stimmten farblich und geschmacklich in allen wesentlichen Parametern überein. Das erfindungsgemäße Verfahren erwies sich damit dem herkömmlichen Verfahren als gleichwertig.

## Patentansprüche

1. Verfahren zur Umrötung von Fleischerzeugnissen unter Verwendung von Mikroorganismen, **dadurch gekennzeichnet, dass** Fleischmasse mit bis zu 1x 10¹² Kolonie bildenden Einheiten pro kg Fleischmasse einer oder mehrerer lebensmitteltauglichen Spezies der Gattungen Staphylococcus oder Kocuria, die in der Lage ist, Arginin in Stickstoffmonoxid zu überführen, und mit bis zu 20 g pro kg Fleischmasse eines Arginin enthaltenden Substrates auf pflanzlicher, tierischer, mikrobieller oder Pilzbasis versetzt wird und einem definierten Umröteprozess unterzogen wird.

2. Verfahren nach Anspruch 1, **dadurch gekennzeichnet, dass** als Substrat ein Hefeextrakt verwandt wird.

3. Verfahren nach Anspruch 1, **dadurch gekennzeichnet, dass** als Substrat ein Gemüsepepton, insbesondere Erbsenpepton verwandt wird.

4. Verfahren nach einem der vorstehenden Ansprüche, **dadurch gekennzeichnet, dass** die Fleischmasse mit 1 bis 10 g Substrat pro kg Fleischmasse versetzt wird.

5. Verfahren nach einem der vorstehenden Ansprüche, **dadurch gekennzeichnet, dass** die Fleischmasse bei einer Temperatur ≤ 60° C Umröten gelassen wird.

6. Verfahren nach einem der vorstehenden Ansprüche **dadurch gekennzeichnet, dass** einer oder mehrere der folgenden Mikroorganismen eingesetzt wird/werden:
Staphylococcus xylosus FASP 2 und FASP 3
Staphylococcus vitulinus FASP 4
Staphylococcus equorum FASP 1 und FASP 5
Kocuria salsicia FASP 6
Kocuria varians FASP 7,
Staphylococcus carnosus FASP 8.

7. Verfahren nach einem der vorstehenden Ansprüche, **dadurch gekennzeichnet, dass** es zur Herstellung von Brühwurst, Brühdauerwurst, Kochwurst und Kochpökelwaren eingesetzt wird.

8. Verfahren nach einem der Ansprüche 1 bis 6, **dadurch gekennzeichnet, dass** es zur Herstellung von Rohwurst und Rohpökelwaren eingesetzt wird.

9. Packungen zur Umrötung von Fleischerzeugnissen, enthaltend einen oder mehrere Stämme einer oder mehrerer Spezies der Gattungen Staphylococcus oder Kocuria, die in der Lage sind, Arginin in Gegenwart eines Substrates auf pflanzlicher, tierischer und mikrobieller Pilzbasis in Stickstoffmonoxid zu überführen, in gefriergetrockneter oder anderweitig konservierter Form sowie, getrennt davon ein Arginin enthaltendes Substrat auf pflanzlicher, tierischer, mikrobieller oder Pilzbasis.

10. Packungen nach Anspruch 9, enthaltend ein Substrat auf pflanzlicher Basis oder Hefebasis.

11. Mikroorganismen der Stämme
Staphylococcus xylosus FASP 2 und FASP 3
Staphylococcus vitulinus FASP 4
Staphylococcus equorum FASP 1 und FASP 5
Kocuria salsicia FASP 6
Kocuria varians FASP 7,
Staphylococcus carnosus FASP 8.
